# EUROPEAN PATENT APPLICATION

(11) **EP 4 843 497 A1**
(43) Date of publication of application: **25.11.2026**
(21) Application number: 25742295.6
(22) Date of filing: 20.01.2025
(51) Int. Cl.: A61B 5/1473, A61B 90/00, A61B 5/145

(54) **TRANSMITTER AND INSERTION DEVICE ASSEMBLY INCLUDING SAME**

(30) Priority: 19.01.2024 KR 20240008852
(71) Applicant: SD Biosensor Inc., Gyeonggi-do 16690 (KR)
(72) Inventor: LEE, Hyo Keun, Suwon-si Gyeonggi-do 16690 (KR); PARK, Hyo-Lim, Suwon-si Gyeonggi-do 16684 (KR); HONG, Soonmin, Hwaseong-si Gyeonggi-do 18477 (KR); KIM, Iek-Lok, Suwon-si Gyeonggi-do 16690 (KR)
(74) Representative: Regimbeau
(86) International application number: PCT/KR2025/001090
(87) International publication number: WO 2025/155147

(57) **Abstract**

Provided is a transmitter of an apparatus for monitoring in-vivo analytes and an insertion device assembly for inserting a sensor of the transmitter into the body of a user and attaching the transmitter externally to the body of the user, in which the transmitter is divided and separated into a sensor assembly and an electronic assembly, thus enabling sterilization of only the sensor assembly without use of a separate shielding device.

## Description

### [Technical Field]

The present disclosure relates to a transmitter of an apparatus for monitoring in-vivo analytes, and an insertion device assembly for inserting a sensor of the transmitter into the body of a user and attaching the transmitter externally to the body of the user.

### [Background Art]

The content described in this section merely provides background information related to the present disclosure and does not constitute the related art.

Recently, as preferences for processed foods have increased, people are more easily exposed to monosaccharides. In this environment, the prevalence of diabetes is also increasing rapidly. When blood glucose levels decrease rapidly or increase rapidly, diabetic patients may suffer from shock, and in rare cases, it may lead to death. For this reason, diabetic patients need to continuously monitor their blood glucose levels.

In the related blood glucose measuring device, a lancet is used to incise the fingertip of a diabetic patient, and the blood emerging from the incision is introduced into a blood glucose analysis device to calculate the blood glucose level.

This type of blood glucose measuring instrument causes pain and fear to the user. In addition, it is practically difficult to make an incision on one's finger every hour to draw blood.

To address these issues, a continuous glucose monitoring (CGM) system has been developed. Once a sensor having a minute thickness and an electronic device (hereinafter, referred to as a "transmitter") for analyzing an analyte (hereinafter, referred to as "blood glucose") measured by the sensor are attached to the skin of a diabetic patient, the blood glucose is continuously measured for a period of one week to ten days.

Since the sensor of the transmitter is very thin, it is difficult to penetrate the skin directly. Accordingly, a configuration (hereinafter, referred to as an "applicator") is required in which the sensor is enclosed by a needle, the sensor and the needle are inserted together, and then the needle is withdrawn.

Meanwhile, according to US 2021-0038131 A1, a user has to insert a sensor introducer 106 into a sensor inserter 500. At this time, the sensor introducer must be aligned and inserted into the sensor inserter, and the user is likely to experience fatigue in this process. Therefore, it is preferable that the applicator and the transmitter of the insertion device assembly be configured as an integrated structure from the time of manufacture.

During an assembly process for integrating the transmitter and the applicator, sterilization is performed, but in the case of the method for sterilizing the applicator using sterilizing gas, the process takes a long time and there is a risk of residual substances remaining.

For this reason, a method of performing sterilization by irradiating an electron beam (e-beam) is widely used when sterilizing medical devices. The e-beam sterilization is a method in which electrons are accelerated and irradiated to collide with an object to be sterilized, and sterilization is performed by the heat generated from the same. This method is advantageous in that rapid sterilization is possible and, unlike the sterilization gas method, no residual substances remain after sterilization, allowing immediate release and distribution without delay after the sterilization.

On the other hand, there is a problem in that electronic components, such as a battery, circuits on the substrate, etc. provided inside the transmitter, may be affected by the electrons irradiated during sterilization.

JP 7210614 B2 discloses a method of using a shield in which, to protect a sensor control device (transmitter), including a sensor control device that incorporates a sensor and electronic components, from the sterilizing e-beam, the sensor control device is shielded at one side of the transmitter and only the sensor to be sterilized is exposed.

This can protect the sensor control device from the sterilizing e-beam, but there is also a problem that a separate shield must be provided, and the e-beam cannot be completely shielded due to the structure of the shield.

### (Prior Art Documents)

US 2021-0038131 A1
JP 7210614 B2

### [Disclosure]

### [Technical Problem]

Accordingly, the present disclosure has been made in view of the problems of the related art described above, and in order to solve the above and other problems, an object is to provide a transmitter capable of sterilizing only a portion that requires sterilization, including a sensor of the transmitter, without a separate shielding device for protecting electronic components of the transmitter from an electron beam (e-beam), and an insertion device assembly including the same.

An object is to provide a transmitter and an insertion device assembly including the same, in which the transmitter is configured to be divided and separated into a sensor assembly and an electronic assembly so that a portion that requires sterilization can be sterilized by only a single e-beam irradiation and a substrate on which electronic components that may risk loss of some functions when exposed to the e-beam are mounted can be protected.

An object is to provide a transmitter capable of quickly and easily performing assembly and sterilization processes through hook or bonding coupling even when the transmitter is configured to be separated into a sensor assembly and an electronic assembly, and an insertion device assembly including the same.

An object is to provide a transmitter capable of preventing damage to the substrate included in the transmitter during sterilization, by configuring a lower surface of the transmitter into two portions, that is, into a base portion and a lower case, and an insertion device assembly including the same.

An object is to provide a transmitter in which the sensor and the substrate can be electrically connected to each other without a separate electrical connecting work, simultaneously upon coupling of the sensor assembly and the electronic assembly, and an insertion device assembly including the same.

An object is to provide a transmitter capable of fixing the sensor to the sensor assembly simultaneously while electrically connecting the sensor, an electrode, and a terminal, and an insertion device assembly including the same.

An object is to provide a transmitter capable of converting a mode of the transmitter and an insertion device assembly including the same.

### [Technical Solution]

In order to achieve the object described above, the present disclosure provides a sensor assembly including a base portion, and a sensor that passes through the base portion, in which a proximal portion of the sensor is positioned at a lower portion of the base portion and a distal portion is positioned at an upper portion of the base portion, and an electronic assembly accommodated in the upper portion of the base portion of the sensor assembly, in which the electronic assembly includes, in a state in which the electronic assembly is coupled to the sensor assembly, a lower case positioned at the upper portion of the base portion, an upper case positioned at an upper portion of the lower case, and a substrate positioned in a space between the upper case and the lower case.

It is preferable that the sensor assembly may further include an electrode portion including a contact terminal positioned close to the sensor side, and a connection terminal electrically connected to the contact terminal, a connector electrically connecting the sensor to the contact terminal, and a sensor housing covering the sensor, the connector, and the contact terminal, and including an opening formed in an upper portion, and the electronic assembly may further include a connection electrode positioned in the lower case to electrically connect the connection terminal to a circuit terminal formed on the substrate.

It is preferable that the connector may include a sensor contact portion in contact with the sensor, and a terminal contact portion extending from the sensor contact portion to contact the contact terminal.

It is preferable that the sensor assembly may further include an installation portion where the connector is installed on the base portion, and the connector is installed such that, in a state where one side of the sensor is supported by one side of the installation portion, the sensor contact portion is in contact with other side of the sensor, thereby fixing the sensor.

It is preferable that the installation portion may further include a hook formed on one side, the connector covers an upper portion of the sensor and includes a plurality of bent portions formed therein, and the connector is fixed to the installation portion by the bent portions being engaged with the hook.

It is preferable that the connector may include a fixing portion including a mounting groove formed therein, wherein a distal portion of the sensor is fitted into the mounting groove, and a connection pin including a sensor contact portion installed on the fixing portion and positioned on the mounting groove side and a terminal contact portion extending from the sensor contact portion to contact the contact terminal.

It is preferable that the connection pin has elasticity, the sensor contact portion of the connection pin is pressed as the distal portion of the sensor is fitted into the mounting groove, and after the sensor is mounted, the sensor contact portion presses the distal portion of the sensor, while the distal portion of the sensor and the contact terminal are electrically connected by the connection pin.

It is preferable that a guide protrusion is further provided, which may be disposed on an inner surface of the sensor housing to guide a position at which the connector is installed.

It is preferable that a holder is further provided, which may be positioned on the base portion to surround a portion of the sensor at a position where the sensor is penetrating, in which an upper portion of the holder closes the opening of the sensor housing.

It is preferable that the sensor housing may further include a conversion terminal formed therein and contacting the contact terminal, and an activation terminal electrically connected to the conversion terminal and exposed to exterior of the sensor housing.

It is preferable that the electronic assembly may further include a conversion terminal formed therein and contacting the substrate, and an activation terminal electrically connected to the conversion terminal and exposed to exterior of the upper case.

An insertion device assembly is provided, which may include a transmitter including a sensor assembly including a base portion, and a sensor that passes through the base portion, in which a proximal portion of the sensor is positioned at a lower portion of the base portion and a distal portion is positioned at an upper portion of the base portion, an electronic assembly attachable to and detachable from the upper portion of the base portion of the sensor assembly, in which the electronic assembly includes, in a state in which the electronic assembly is coupled to the sensor assembly, a lower case positioned at the upper portion of the base portion, an upper case positioned at an upper portion of the lower case, and a substrate positioned in a space between the upper case and the lower case, and an applicator having an internal space in which the transmitter is mounted, the applicator including an upper housing, a lower housing coupled to a lower portion of the upper housing and forming an internal space with the housing, a needle assembly including a grip portion positioned above the transmitter in the internal space, and a needle positioned below the grip portion to pass through the transmitter and surround a portion of a circumference of a proximal portion of the sensor, a needle carrier disposed in the internal space and configured to linearly move the needle assembly, and a driving portion that provides power to linearly move the needle assembly.

It is preferable that the sensor assembly may further include an electrode portion including a contact terminal positioned close to the sensor side, and a connection terminal electrically connected to the contact terminal, a connector electrically connecting the sensor to the contact terminal, and a sensor housing covering the sensor, the connector, and the contact terminal, and including an opening formed in an upper portion, and the electronic assembly may further include a connection electrode positioned in the lower case to electrically connect the connection terminal to a circuit terminal formed on the substrate.

It is preferable that the connector may include a sensor contact portion in contact with the sensor, and a terminal contact portion extending from the sensor contact portion to contact the contact terminal.

It is preferable that the sensor assembly may further include an installation portion where the connector is installed on the base portion, and the connector is installed such that, in a state where one side of the sensor is supported by one side of the installation portion, the sensor contact portion is in contact with other side of the sensor, thereby fixing the sensor.

It is preferable that the installation portion may further include a hook formed on one side, the connector covers an upper portion of the sensor and includes a plurality of bent portions formed therein, and the connector is fixed to the installation portion by the bent portions being engaged with the hook.

It is preferable that the connector may include a fixing portion including a mounting groove formed therein, wherein a distal portion of the sensor is fitted into the mounting groove, and a connection pin including a sensor contact portion installed on the fixing portion and positioned on the mounting groove side and a terminal contact portion extending from the sensor contact portion to contact the contact terminal.

It is preferable that the connection pin has elasticity, the sensor contact portion of the connection pin is pressed as the distal portion of the sensor is mounted in the mounting groove, and after the sensor is mounted, the sensor contact portion presses the distal portion of the sensor, while the distal portion of the sensor and the contact terminal are electrically connected by the connection pin.

It is preferable that a guide protrusion is further provided, which may be disposed on an inner surface of the sensor housing to guide a position at which the connector is installed.

It is preferable that a holder is further provided, which may be positioned on the base portion to surround a portion of the sensor at a position where the sensor is penetrating, in which an upper portion of the holder closes the opening of the sensor housing, and the needle passes through the holder.

It is preferable that a protrusion is further provided, which may be positioned on a lower portion of the grip portion and pass through the opening of the sensor housing to surround a portion of the sensor.

It is preferable that a sealing member is further provided, which may be positioned between the protrusion and the opening of the sensor housing, in which, in a state in which the needle assembly is coupled to the transmitter, an internal space of the grip portion and the sensor housing is sealed by the sealing member.

It is preferable that the sensor housing may further include a conversion terminal formed therein and contacting the contact terminal, and an activation terminal electrically connected to the conversion terminal and exposed to exterior of the sensor housing, and the applicator may further include a conductor that contacts the activation terminal while the transmitter is in a mounted state.

It is preferable that the electronic assembly may further include a conversion terminal formed therein and contacting the substrate, and an activation terminal electrically connected to the conversion terminal and exposed to exterior of the upper case, and the applicator may further include a conductor that contacts the activation terminal while the transmitter is in a mounted state.

### [Advantageous Effects]

With the transmitter and the insertion device assembly including the same according to the present disclosure, the following effects can be obtained.
1) First, by configuring the transmitter to be divided and separated into the sensor assembly and the electronic assembly, it is possible to sterilize only the sensor assembly without a separate shielding device.
2) By configuring the transmitter to be divided and separated into the sensor assembly and the electronic assembly, the sensor and needle, which are parts directly inserted into the user's body, and the lower surface of the transmitter and the lower surface of the applicator, which contact the user's skin, are modularized as the first assembly, thereby enabling sterilization of required portions with only one e-beam irradiation.
3) By configuring the transmitter to be divided and separated into the sensor assembly and the electronic assembly, the substrate, on which the electronic components that may risk partial functional loss when directly exposed to e-beam irradiation are mounted, and structures such as the upper housing or the driving portion of the applicator, which substantially require little sterilization, are excluded from the first assembly, and accordingly, there is an advantage in that sterilization is performed by e-beam irradiation without risk of malfunction of the electronic devices mounted on the substrate and sterilization of the insertion device assembly can be achieved within a narrow range and short e-beam irradiation time.
4) After sterilization of the sensor assembly, the electronic assembly can be easily coupled to the sensor assembly using a simple method such as hook structure, bonding, or the like, so that assembly and sterilization processes can be performed quickly and easily even when the transmitter is configured to be divided and separated into the sensor assembly and the electronic assembly.
5) By configuring the transmitter to be divided and separated into the sensor assembly and the electronic assembly, the lower surface of the transmitter includes two parts, namely, the lower surface of the base portion and the lower surface of the lower case, so that force applied to the substrate accommodated in the transmitter upon firing through the applicator is dispersed and reduced.
6) By arranging the terminals and the electrodes to electrically connect the sensor assembly and the electronic assembly during assembly, the sensor and the substrate can be electrically connected simultaneously with coupling of the sensor assembly and the electronic assembly, without requiring a separate electrical connection work.
7) By configuring the connector that connects the electrodes and the terminals to the sensor in a clip form, the sensor can be electrically connected to the electrodes and the terminals using the connector without a separate configuration for fixing the sensor, and the sensor can also be fixed to the sensor assembly.
8) In addition, by installing the conversion terminal and the activation terminal, it is possible to convert the activation of the operation of the transmitter by separating the transmitter from the applicator.

### [Brief Description of the Drawing]

FIG. 1 is an overall perspective view of a transmitter according to an embodiment of the present disclosure.
FIG. 2 is an exploded perspective view of the transmitter according to an embodiment of the present disclosure, illustrating a state in which a sensor assembly and an electronic assembly are separated from each other.
FIG. 3 is an exploded perspective view of the sensor assembly of the transmitter according to an embodiment of the present disclosure.
FIG. 4 is an enlarged view of a portion of the exploded perspective view of the sensor assembly according to an embodiment of the present disclosure.
FIG. 5 is a perspective view illustrating an electrode portion of the sensor assembly according to an embodiment of the present disclosure.
FIG. 6 is an enlarged view of a portion of the exploded perspective view of the sensor assembly according to an embodiment of the present disclosure.
FIG. 7 is a cross-sectional view of one side of a sensor housing of the sensor assembly according to an embodiment of the present disclosure.
FIG. 8 is a bottom view of the sensor housing according to an embodiment of the present disclosure.
FIG. 9 is an exploded perspective view of the electronic assembly of the transmitter according to an embodiment of the present disclosure.
FIG. 10 is a partial bottom view of a lower case of the electronic assembly according to an embodiment of the present disclosure.
FIG. 11 is a perspective view illustrating a state in which a needle assembly of an applicator is coupled to the sensor assembly according to an embodiment of the present disclosure.
FIG. 12 is a perspective view illustrating a state in which the electronic assembly is additionally coupled after the needle assembly of the applicator is coupled to the sensor assembly according to an embodiment of the present disclosure.
FIG. 13 is a perspective view illustrating a method for assembling a sensor insertion assembly including the transmitter and the applicator according to an embodiment of the present disclosure.
FIG. 14 is an exploded perspective view of a transmitter according to another embodiment of the present disclosure, illustrating a state in which the sensor assembly and the electronic assembly are separated from each other.
FIG. 15 is an exploded perspective view of a sensor assembly of the transmitter according to another embodiment of the present disclosure.
FIG. 16 is an enlarged view of a portion of the exploded perspective view of the sensor assembly according to another embodiment of the present disclosure.
FIG. 17 is a longitudinal cross-sectional view of a connector according to another embodiment of the present disclosure.
FIG. 18 is a bottom view of a sensor housing according to another embodiment of the present disclosure.
FIG. 19 is an exploded perspective view of an upper case part of an electronic assembly of the transmitter according to another embodiment of the present disclosure.
FIG. 20 is a partial bottom view of a lower case of the electronic assembly according to another embodiment of the present disclosure.
FIG. 21 is a longitudinal cross-sectional view illustrating a state in which the transmitter and a needle assembly are separated according to another embodiment of the present disclosure.
FIG. 22 is a longitudinal cross-sectional view illustrating a state in which the transmitter and the needle assembly are coupled to each other according to another embodiment of the present disclosure.

### [Mode for Carrying Out the Invention]

Hereinafter, some embodiments of the present disclosure will be described in detail with reference to example drawings. It should be noted that reference numerals are given to the elements shown in the drawings such that the same elements, whether they are illustrated in the same drawing or different drawings, will be denoted by the same reference numerals. In addition, in the description of the present disclosure, the detailed description of the related well-known configurations or functions will be omitted if it is determined that it may obscure the subject matter of the present disclosure.

In the description of the elements of an embodiment according to the present disclosure, modifiers or labels such as first, second, i), ii), a), and b) may be used. These modifiers or labels are used solely for the purpose of distinguishing an element from another element, without limiting the nature, sequence or order of the corresponding elements. In the description, when a portion "comprises" or "includes" an element, this means that it may further include yet another element, rather than excluding the other elements, unless otherwise explicitly stated to the contrary.

In the following description, an "insertion device assembly" refers to an assembly in which an applicator and a transmitter are assembled.

In addition, "distal position" refers to a position relatively farther away from the skin than "proximal position". Meanwhile, the distal position and the proximal position are relative positional concepts, so an element can be understood as being proximal to another element while at the same time distal.

In addition, "fire" refers to an action of a user activating the applicator so that the needle is inserted into the user's skin.

In addition, "retraction" refers to the process in which a needle, after being inserted through the user's skin, returns to its original position.

In addition, "upward" refers to the direction from the proximal position to the distal position.

In addition, "downward" refers to the direction from the distal position to the proximal position.

In addition, "horizontal" refers to a plane that the applicator contacts when it is used, and may also refer to a surface of the skin. In the present disclosure, the curvature of the skin surface is not considered, and the description is made under the assumption that the user's skin surface is a flat surface.

### 1. Configuration of transmitter

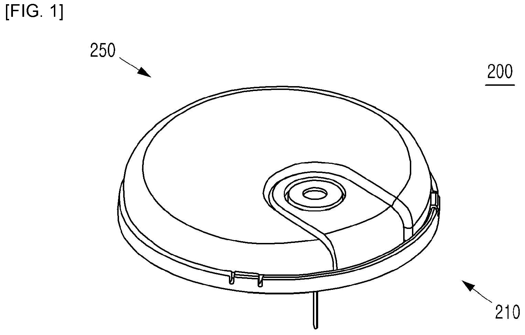

Hereinafter, the configuration of a transmitter 200 of an insertion device assembly according to an embodiment of the present disclosure will be described in detail with reference to FIGS. 1 to 10 appended hereto.

The transmitter 200 includes a sensor 214 formed with a fine thickness and inserted into the user's skin, and electronic components necessary to analyze and transmit analytes measured by the sensor 214.

According to an embodiment of the present disclosure, the transmitter 200 includes a sensor assembly 210 and an electronic assembly 250 coupled to an upper portion of the sensor assembly 210.

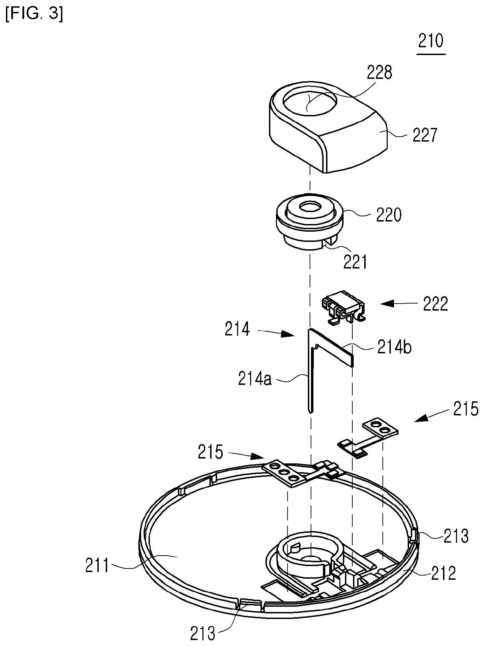

The configuration of the sensor assembly 210 will be described in further detail with reference to FIG. 3.

The sensor assembly 210 includes a base portion 211, the sensor 214, a holder 220, an electrode portion 215, a connector 222, and a sensor housing 227.

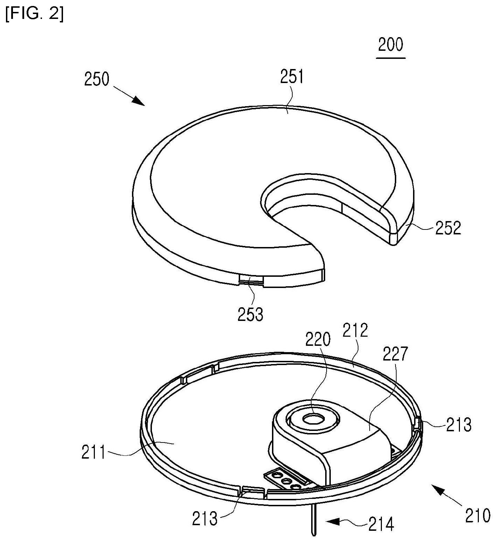

The base portion 211 is formed in a plate shape. In FIGS. 2 to 3, the base portion 211 configured in a circular plate shape is illustrated, but aspects are not limited thereto.

The base portion 211 includes an outer wall 212 extending upward by a predetermined distance from a circumferential side thereof, and a portion of the outer wall 212 may be configured as a fastening hook 213.

A through-hole is formed in one side of the base portion 211 to allow a proximal portion 214a of the sensor 214 to pass through and be positioned.

The sensor 214 is positioned on the base portion 211. Specifically, the proximal portion 214a of the sensor 214 is positioned to pass through the through-hole of the base portion 211 and be exposed below the base portion 211, and a distal portion 214b of the sensor 214 extends from the proximal portion 214a and is positioned at an upper side of the base portion 211.

The holder 220 is positioned above the sensor 214 on the base portion 211. The holder 220 is positioned to surround a portion of the upper portion of the sensor 214 at a region where the sensor 214 passes through the through-hole. Specifically, a portion between the proximal portion 214a and the distal portion 214b of the sensor 214 is inserted into a groove 221 formed in a lower portion of the holder 220.

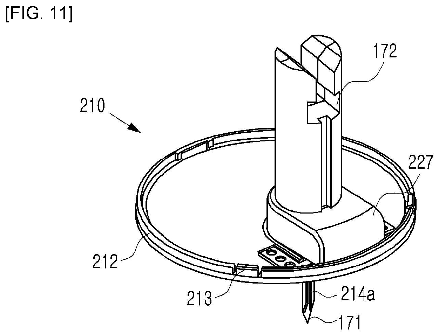

The holder 220 includes an elastic material at least in an upper portion so that a needle 171 of the applicator may pass through an upper surface of the holder 220 (see FIG. 11), and after attachment to the user's body, when the needle 171 is withdrawn, the penetrated portion may be closed by elasticity.

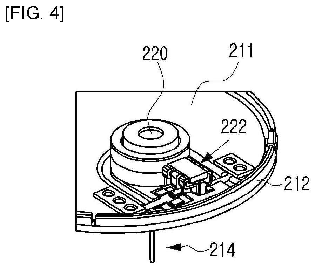

The configuration of the electrode portion 215 and the connector 222 will be described in further detail with reference to FIGS. 4 to 6.

The electrode portion 215 is positioned on both sides of the sensor 214, at one side of the base portion 211. The electrode portion 215 includes contact terminals 216 and connection terminals 217.

The contact terminals 216 are positioned close to both sides of the sensor 214, respectively, the connection terminals 217 extend from the contact terminals 216 to the other sides, and the contact terminals 216 and the connection terminals 217 are electrically connected to each other.

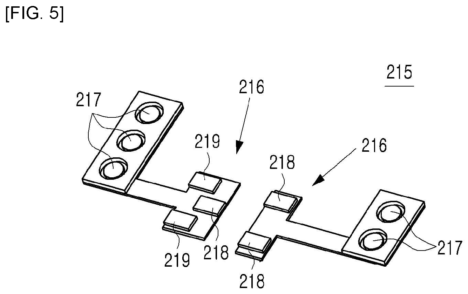

As illustrated in FIG. 5, a plurality of contact terminals 216 may be formed, and the connection terminals 217 may include a plurality of connection terminals 217, each being electrically connected to the plurality of contact terminals 216.

FIGS. 4 to 6 illustrate two groups of contact terminals 216, one including three contact terminals and the other including two contact terminals 216, on both sides of the sensor 214, and two corresponding groups of connection terminals 217, one including three connection terminals 217 and the other including two connection terminals 217 and electrically connected to the contact terminals 216, but aspects are not limited thereto.

Some of the plurality of contact terminals 216 may be configured as internal terminals 218 electrically connected to the sensor 214 by the connector 222, and the others may be external terminals 219 connected by a conversion terminal 229 to be described below.

FIG. 5 illustrates that, among the five contact terminals 216, two contact terminals 216 of the group of three are disposed as the external terminals 219 and the remaining one as the internal terminal 218, but aspects are not limited thereto.

The connector 222 is positioned on the base portion 211 and electrically connects the sensor 214 to the contact terminal 216.

The connector 222 includes a sensor contact portion 223 and a terminal contact portion 224, and the sensor contact portion 223 and the terminal contact portion 224 include a plurality of contact portions extending from a center of the connector 222 and connected.

The sensor contact portion 223 is positioned in contact with one side of the sensor 214, and the terminal contact portion 224 is in contact with the contact terminal 216, thereby electrically connecting the sensor 214 and the contact terminal 216.

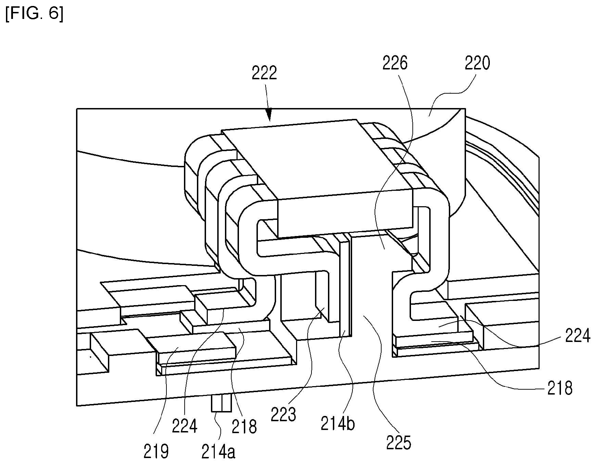

As illustrated in FIG. 6, the base portion 211 includes an installation portion 225 that is positioned on and protrudes from one side, and configured such that, when one side of the sensor 214 is supported on one side of the installation portion 225, the sensor contact portion 223 contacts the other side of the sensor 214, thus securing the sensor 214 between the sensor contact portion 223 and the installation portion 225.

In addition, the connector 222 includes bent portions formed as the sensor contact portion 223 and the terminal contact portion 224 extend and are bent. That is, it is configured in the form of a clip that may be inserted into the installation portion 225.

The installation portion 225 includes an installation hook 226 that is positioned on and protrudes from one side, and the connector 222 may be securely installed in the installation portion 225 as the bent portion is engaged with the installation hook 226 while the connector 222 is inserted in the installation portion 225.

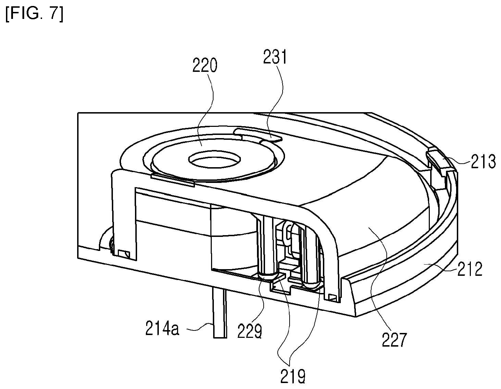

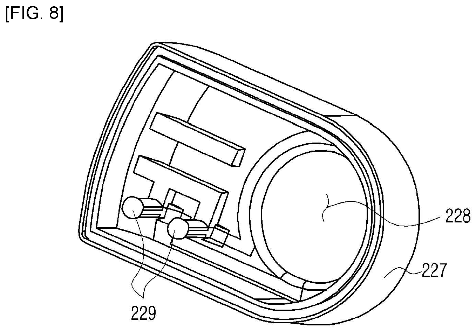

The configuration of the sensor housing 227 will be described further with reference to FIGS. 7 and 8.

The sensor housing 227 is positioned to cover an upper portion of the base portion 211. Rather than covering the entire upper portion of the base portion 211, the sensor housing 227 is configured to cover one side of the base portion 211, and specifically, is positioned to cover upper portions of the sensor 214, the connector 222, and the contact terminal 216 from above the base portion 211.

As illustrated in FIGS. 3 and 7, an opening 228 is formed in one side of an upper surface of the sensor housing 227 such that the upper surface of the holder 220 is exposed through the opening 228 when the sensor 214 is positioned to cover the upper portions of the sensor 214, the connector 222, and the contact terminal 216.

The sensor housing 227 includes the conversion terminal 229 and an activation terminal 231.

The conversion terminal 229 extends inside the sensor housing 227, and has a proximal portion in contact with a portion of the contact terminal 216, that is, with the external terminal 219.

The activation terminal 231 extends to the other side of the conversion terminal 229 and is exposed to the outside of the sensor housing 227, and the activation terminal 231 comes into contact with or is released from contact with the conductor (not illustrated) included in the applicator according to the operation of the applicator to be described below.

Specifically, with the transmitter 200 being installed in the applicator, before firing, the conductor and the activation terminal 231 are in contact with each other, in which case the operation of the transmitter 200 is deactivated, and by the firing of the applicator, the transmitter 200 is attached to the user's body and separated from the applicator, thereby releasing the contact between the conductor and the activation terminal, so that the operation of the transmitter 200 is activated.

In addition, although not illustrated, the base portion 211 may include all or part of an adhesive tape on a lower surface, and the transmitter 200 may be attached to the user's skin by the adhesive tape when the sensor 214 is inserted in the user's body.

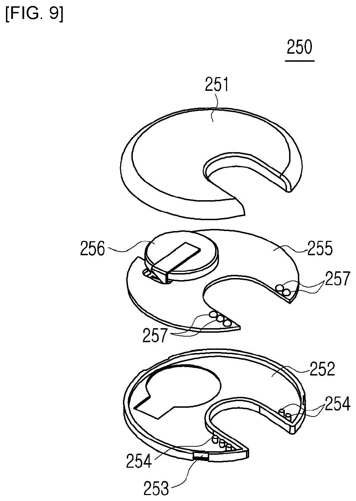

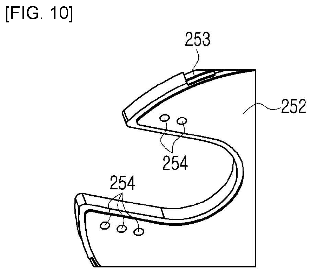

Next, the configuration of the electronic assembly 250 of the transmitter 200 will be described with further reference to FIGS. 9 and 10.

The electronic assembly 250 is attachable to and detachable from the upper portion of the base portion 211 of the sensor assembly 210, and the electronic assembly 250 includes a lower case 252, an upper case 251, and a substrate 255.

The upper case 251 and the lower case 252 are vertically coupled to each other to form an external appearance of the electronic assembly 250, and there is a predetermined accommodation space formed between the upper case 251 and the lower case 252 coupled to each other.

The upper case 251 and the lower case 252 may have a shape corresponding to the shape of the base portion 211 of the sensor assembly 210, but one side thereof may be open in a shape corresponding to the shape of the sensor housing 227 so that the electronic assembly 250 may be positioned side by side with the sensor housing 227 when coupled to the upper portion of the base portion 211.

The substrate 255 is positioned inside the space formed between the upper case 251 and the lower case 252.

The substrate 255 is preferably formed in a shape corresponding to the upper case 251 and the lower case 252, that is, has one side open, so as to be positioned in the space between the upper case 251 and the lower case 252.

The substrate 255 has, mounted thereon, electronic components for analyzing a concentration of an analyte within the user's body sensed by the sensor 214 and transmitting information related to the concentration to an external device, and a battery 256 for supplying power to the electronic components.

The substrate 255 is not limited to any specific type and may be configured, for example, as a printed circuit board (PCB).

Circuit terminals 257 are positioned on one side of the substrate 255 to transmit a signal of the analyte measured by the sensor 214 or a mode conversion signal from the outside to each of the electronic components on the substrate 255, and the circuit terminals 257 are equal in number to the contact terminals 216.

Connection electrodes 254 are positioned on one side of the lower case 252, that is, at locations where the circuit terminals 257 of the substrate 255 are positioned when the substrate 255 is in the accommodated state, and are in contact with the circuit terminals 257 of the substrate 255.

In addition, recessed locking portions 253 are formed on a circumferential side of the lower case 252, and the locking portions 253 are formed at positions and in numbers corresponding to the fastening hooks 213 positioned on the base portion 211.

When the electronic assembly 250 is coupled to the sensor assembly 210, a portion of the lower case 252 of the electronic assembly 250 is inserted into the outer wall 212 of the base portion 211, and the fastening hooks 213 of the base portion 211 are hooked in the locking portions 253 of the lower case 252 so that the electronic assembly 250 is coupled to the sensor assembly 210.

When the electronic assembly 250 is coupled to the sensor assembly 210, the substrate 255 positioned inside the electronic assembly 250 is disposed on the lower case 252, and accordingly, positioned higher than a predetermined height of the outer wall 212 of the base portion 211, higher than the upper portion of the outer wall 212.

That is, since the lower case 252 of the electronic assembly 250 and the base portion 211 are overlapped and disposed on the lower portion of the substrate 255, the impact applied to the substrate 255 can be absorbed, and the substrate 255 can be accommodated in the transmitter 200 more stably.

When the sensor assembly 210 and the electronic assembly 250 are coupled to each other, the analyte signal measured by the sensor 214 is transmitted to the electronic components mounted on the substrate 255 through the connector 222, the contact terminals 216, the connection terminals 217, the connection electrodes 254, and circuit electrodes.

### 2. Configuration of applicator

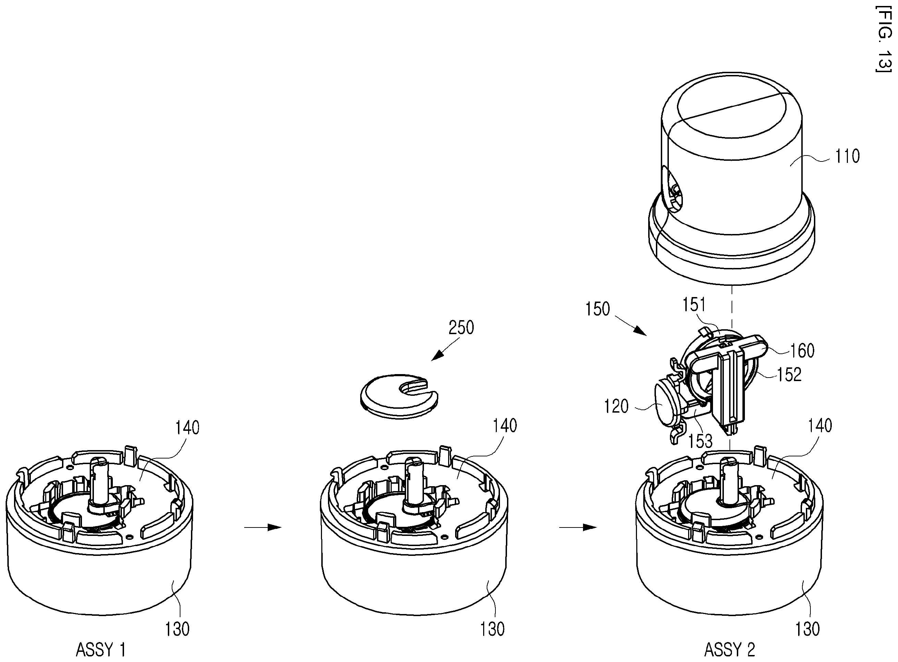

The configuration of the applicator will be described with reference to FIGS. 11 to 13.

The applicator includes an upper housing 110, a lower housing 140, a needle assembly, and a driving portion 150.

The upper housing 110 is configured to be grasped by the user and preferably has an approximately cylindrical shape, and a lower side of the upper housing 110 is open.

The lower housing 140 is configured such that an internal space is formed as the lower housing 140 is coupled to a lower portion of the upper housing 110, and a lower side of the lower housing 140 is disposed inside a cap 130.

The transmitter 200 is mounted in the internal space formed by the coupling of the lower housing 140 to the lower portion of the upper housing 110.

In this state, as described above, the conductor (not illustrated) installed in the applicator is in contact with the activation terminal of the transmitter, and therefore, the operation of the transmitter is in an inactive state.

When the applicator is in use, detaching the cap 130 from the lower housing 140 brings the insertion device assembly into a state in which it can be used by a user, and a lower surface of the lower housing 140 is in direct contact with the user's skin for use.

The needle assembly includes a grip portion 172 and the needle 171. In the internal space formed by coupling the lower housing 140 to the lower portion of the upper housing 110, a portion of the needle assembly passes through the holder 220 of the transmitter 200.

The grip portion 172 is positioned above the holder 220 of the transmitter 200. In a state where the lower portion of the grip portion 172 is disposed above the holder 220, the needle 171 extends from the lower portion of the grip portion 172, passes through the holder 220 and then the lower through-hole formed in the base portion 211 of the transmitter 200, and is positioned below the lower surface of the transmitter 200.

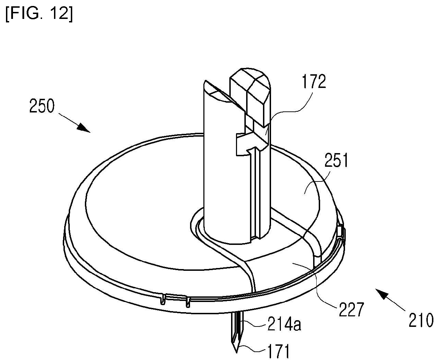

As illustrated in FIGS. 11 and 12, when the needle 171 passes through the transmitter 200, the needle 171 is positioned to surround at least a portion of the circumference of the proximal portion 214a of the sensor 214 disposed in the transmitter 200.

The driving portion 150 is disposed above the transmitter 200 in the internal space formed by the upper housing 110 and the lower housing 140.

The driving portion 150 is configured to linearly move the transmitter 200 and the needle assembly along a longitudinal direction of the applicator. The driving portion 150 includes a spring 151, a wheel 152, and a locking member 153.

The spring 151 serves as a power source that provides power to a needle carrier 160 so that the needle carrier 160, which will be described below, can be linearly moved.

The spring 151 is preferably a wound reel spring 151. The spring 151 is assembled in a compressed state initially.

In order to release compression of the spring 151, a button 120, penetrating inward from an outer circumferential surface of the upper housing 110, may be provided.

As the fastening portion securing the compression of the spring 151 is released by the pressure of the button 120, the spring 151 may be tensioned to return to its original state, thereby causing one end of the spring 151 to rotate. That is, the principle of a kind of Scotch Yoke or double slider crank mechanism, in which the rotational motion of the spring 151 is converted into linear motion of the needle carrier 160 and the needle assembly, may be applied.

The wheel 152, having one side surface fastened to one end of the spring 151, is configured to rotate together with the spring 151 by the rotation of the spring 151. A groove for coupling with the locking member 153 may be formed on an outer circumferential surface of the wheel 152.

The locking member 153 has one end coupled to the button 120 and the other end fastened to the wheel 152. In this case, the locking member 153 includes a groove formed in the other end to be engaged with the groove of the wheel 152. Accordingly, the rotation of the wheel 152 is prevented, and furthermore, the tension of the spring 151 is prevented.

When the button 120 is pressed by the user and moved toward the interior of the applicator, the locking member 153 is also moved in conjunction with the button 120. As a result, fastening between the other end of the locking member 153 and the groove of the wheel 152 is released, and the wheel 152 may rotate together due to tension and rotation caused by the elasticity of the spring 151.

The needle carrier 160 is configured to linearly move inside the upper housing 110 and the lower housing 140.

In this case, the linear movement of the needle carrier 160 may be induced by the rotational motion of the driving portion 150. To this end, it is preferable that the needle carrier 160 has an overall shape of a letter T or a cross.

One side of the needle carrier 160 is coupled to the other side of the wheel 152, and a lower end is coupled to an upper portion of the grip portion 172 of the needle assembly, so that the linear movement of the needle assembly may be performed according to the rotational movement of the wheel 152 according to the tension of the spring 151.

Specifically, according to the firing operation of the applicator triggered upon pressing of the button 120, the needle assembly and the transmitter 200 are moved downward together so that the needle 171 and the sensor 214 are inserted into the user's body, and after the lower surface of the transmitter 200 is attached and fixed to the user's body, the needle carrier 160 is moved upward so that only the needle assembly is separated from the transmitter 200.

At this time, contact between the conductor (not illustrated) installed in the applicator and the activation terminals of the transmitter is released, and the operation of the transmitter is activated.

### 3. Transmitter and applicator according to another embodiment

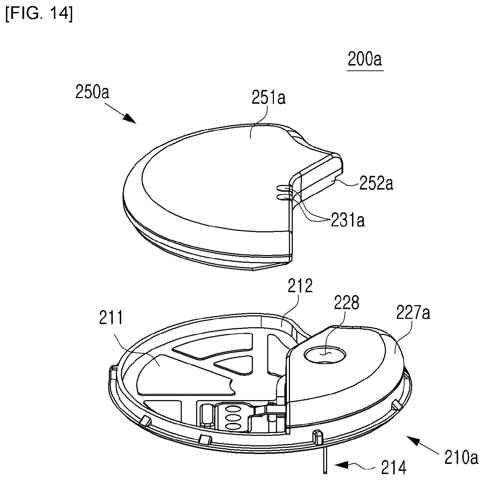

Next, the transmitter 200a and the applicator according to another embodiment of the present disclosure will be described with reference to FIGS. 14 to 22.

In the description of the following embodiment, differences of the transmitter 200a and the applicator from those described above will be focused, and the same configuration will not be described in detail.

In addition, each configuration of the transmitter 200a and the applicator according to another embodiment may be alternatively applied to the embodiment described above.

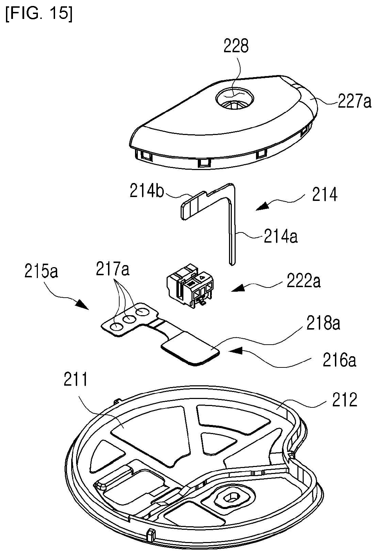

As illustrated in FIGS. 14 and 15, the transmitter 200a according to another embodiment does not include a holder.

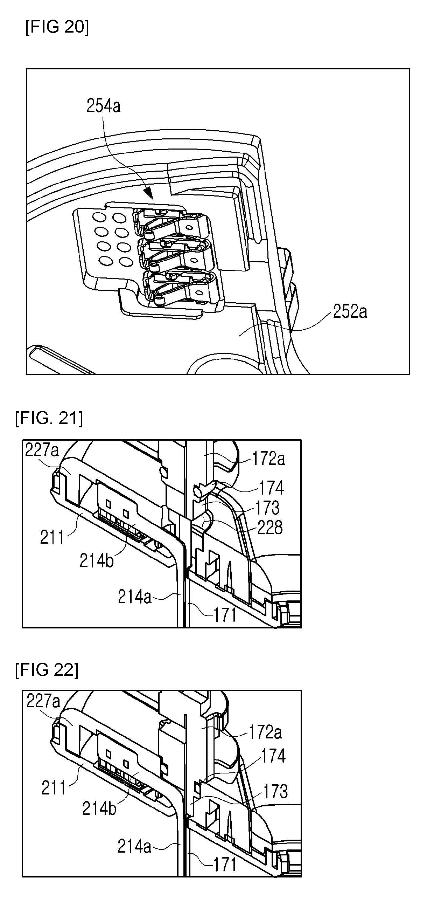

Therefore, the opening 228 formed in the sensor housing 227a of the sensor assembly 210a is not closed by the holder, but the opening 228 is closed by a protrusion 173 formed on the lower portion of the grip portion 172a of the needle assembly of the applicator, and further, when the needle 171 passes through the transmitter 200, the grip portion 172a may be stably aligned with the transmitter 200 (see FIGS. 21 and 22).

In addition, a sealing member 174 is positioned between the protrusion 173 and the opening 228 of the sensor housing 227a, and the sealing member 174 may be configured as a ring-shaped member having elasticity.

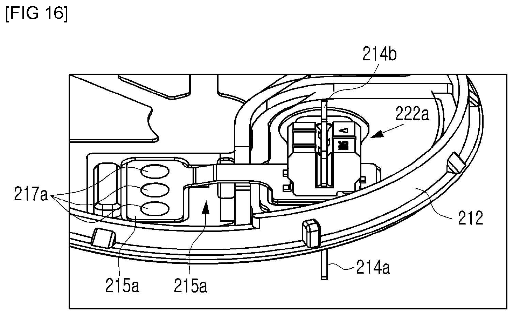

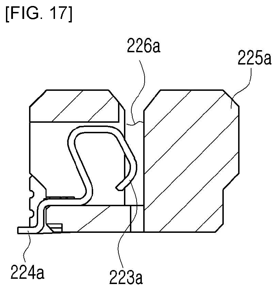

The configuration of an electrode portion 215a and a connector 222a according to another embodiment will be described in detail with reference to FIGS. 16 and 17.

In another embodiment, the electrode portion 215a is positioned on one side of the sensor 214 on the base portion 211. The electrode portion 215a includes a contact terminal 216a and a connection terminal 217a.

The contact terminal 216a does not include an external terminal, but includes an internal terminal 218a electrically connected to the sensor 214.

The contact terminal 216a is positioned below a distal portion 214b of the sensor 214, the connection terminal 217a extends from the contact terminal 216a to the other side, and the contact terminal 216a and the connection terminal 217a are electrically connected to each other.

The connector 222a is positioned on the base portion 211 and electrically connects the sensor 214 to the contact terminal 216a.

The connector 222a includes a fixing portion 225a and a connection pin.

The fixing portion 225a is positioned on the base and above the contact terminal 216a, and a mounting groove 226a is formed in the fixing portion 225a.

The connection pin is installed at the fixing portion 225a and includes a sensor contact portion 223a and a terminal contact portion 224a.

The sensor contact portion 223a is positioned on one side of the connection pin, at the mounting groove 226a side. The terminal contact portion 224a extends from the sensor contact portion 223a and is positioned in contact with the contact terminal 216a.

The connection pin is formed of a conductive material in a bent configuration to provide elasticity, and a plurality of connection pins may be installed on both sides with respect to the mounting groove 226a.

In a state in which the connector 222a is installed on the contact terminal 216a, the distal portion 214b of the sensor 214 is fitted into the mounting groove 226a above the fixing portion 225a, thereby pressing the sensor contact portion 223a into contact with the sensor, and after the sensor is mounted in the mounting groove 226a, the sensor contact portion 223a presses against the distal portion 214b of the sensor 214 so that the distal portion 214b of the sensor 214 and the contact terminal 216a are electrically connected by the connection pin, and the distal portion 214b of the sensor 214 is also mechanically fixed within the mounting groove 226a.

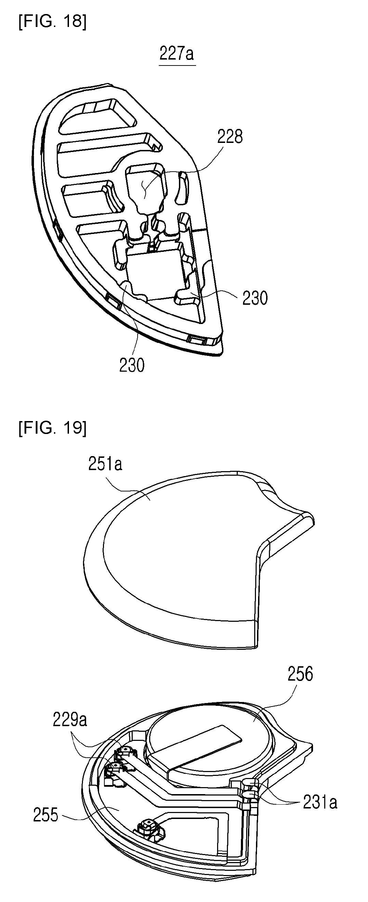

The configuration of the sensor housing 227a will be described with reference to FIG. 18.

According to another embodiment, the sensor housing 227a does not include a conversion terminal 229a nor an activation terminal 231a extending from the conversion terminal 229a to exterior of the sensor housing 227a.

According to another embodiment, a guide protrusion 230 is disposed inside the sensor housing 227a, and the guide protrusion 230 is disposed with a space into which the upper portion of the connector 222a can be fitted, thereby guiding the position at which the connector 222a is installed during assembly of the sensor housing 227a.

Next, the configuration of an electronic assembly 250a according to another embodiment will be described with reference to FIGS. 19 and 20.

A circuit terminal for transmitting a signal of an analyte measured by the sensor 214 or a mode conversion signal from the outside to each of the electronic components on the substrate 255 and a connection electrode 254a in contact with the circuit terminal are positioned on the substrate 255 of the electronic assembly 250a, at a location corresponding to the connection terminal 217a of the base portion 211.

In addition, the electronic assembly 250a according to another embodiment includes the conversion terminal 229a and the activation terminal 231a.

The conversion terminal 229a extends inside a space formed by the upper case 251a and the lower case 252a of the electronic assembly 250a and is connected to the substrate 255.

The activation terminal 231a extends to the other side of the conversion terminal 229a and is exposed to the outside of the upper case 251a.

In this case, a conductor (not illustrated) is positioned in the applicator at a location corresponding to the activation terminal 231a positioned in the upper case 251a, so that, as described above in the previous embodiment, when the transmitter 200a installed in the applicator is in the state prior to firing, the conductor and the activation terminal 231a are in contact with each other, whereby the operation of the transmitter 200a is deactivated, and upon the firing operation of the applicator, the transmitter 200a is attached to the user's body and separated from the applicator, and thus released from the contact and activated.

### 4. Method for assembling insertion device assembly

Next, a method for sterilizing and assembling the insertion device assembly according to an embodiment of the present disclosure will be described by referring back to the accompanying drawings.

First, the insertion device assembly is divided into a first assembly (ASSY 1) state and a second assembly (ASSY 2) state according to assembly order.

The first assembly (ASSY 1) includes a part of the transmitter 200 and a part of the applicator, and the second assembly (ASSY 2) further includes the remaining part of the transmitter 200 and the remaining part of the applicator in the first assembly (ASSY 1).

First, as illustrated in FIGS. 3, 11, and 13, the first assembly (ASSY 1) is assembled.

The base portion 211, the electrode portion 215, the sensor 214, the holder 220, the connector 222, and the sensor housing 227 of the sensor assembly 210 are assembled, the needle 171 passes through the holder 220 and the grip portion 172 is positioned above the holder 220 so that the needle assembly is assembled, and the sensor housing 227 and the needle assembly are mounted together at an upper portion of the internal space of the lower housing 140 of the applicator. By coupling the cap 130 to the lower housing 140, the first assembly (ASSY 1) is assembled.

In addition, as in the case of another embodiment described above, when the transmitter 200a is not provided with the holder 220, the base portion 211, the electrode portion 215a, the sensor 214, the connector 222a, and the sensor housing 227a of the sensor assembly 210a are assembled, the needle 171 passes through the transmitter 200a via the opening 228 of the sensor housing 227a, and the protrusion 173 of the grip portion 172a of the needle assembly is inserted into the opening 228 of the sensor housing 227a and positioned at the upper portion, so that the first assembly (ASSY 1) is assembled.

Then, e-beam is irradiated to the assembled first assembly (ASSY 1), thereby sterilizing the first assembly (ASSY 1).

As described above, the sensor 214 and the needle 171 are inserted directly into the user's body, and the lower surface of the transmitter 200 and the lower surface of the applicator, which are portions contacting the user's skin, require sterilization.

After the sterilization of the first assembly (ASSY 1), the second assembly (ASSY 2) is assembled.

The second assembly (ASSY 2) is assembled by assembling the remaining part of the transmitter 200 and the remaining part of the applicator with the first assembly (ASSY 1).

As illustrated in FIGS. 9, 11, 12, and 13, for the second assembly (ASSY 2), the electronic assembly 250 is assembled such that the substrate 255 is accommodated between the upper case 251 and the lower case 252, and the assembled electronic assembly 250 is coupled to the upper portion of the sensor assembly 210 of the first assembly (ASSY 1).

At this time, the electronic assembly 250 and the sensor assembly 210 are coupled with each other as the fastening hook 213 formed on the base portion 211 of the sensor assembly 210 is caught in the locking portions 253 formed in the lower case of the electronic assembly 250, and after the electronic assembly 250 and the sensor assembly 210 are coupled, UV bonding is performed between the interior of the outer wall 212 of the sensor assembly 210 and the lower case 252 of the electronic assembly 250.

Although the embodiment is described in which the lower case 252 of the electronic assembly 250 and the base portion 211 of the sensor assembly 210 are coupled by a hook structure, aspects are not limited thereto, and the lower case 252 and the base portion 211 of the sensor assembly 210 may be coupled by bonding using only an adhesive.

In addition, the second assembly (ASSY 2) is assembled by fitting a lower end of the needle carrier 160 in the grip portion 172 of the needle assembly, coupling the driving portion 150 to one side of the needle carrier 160, and covering and coupling the upper housing 110 provided with the button 120 onto an upper portion of the lower housing 140.

Then, a process of packaging the assembled second assembly (ASSY 2) is further included, and at this time, the conductor (not illustrated) installed in the applicator is in contact with an activation terminal 231 of the transmitter 200.

According to an embodiment of the present disclosure, a transmitter holder (not illustrated) may be further included. The transmitter holder (not illustrated) may be positioned to cover the upper portion of the transmitter 200 and surround the grip portion 172. A conductor may be installed in a transmitter holder (not illustrated) so as to contact the activation terminal 231 of the transmitter 200 during assembly of the second assembly (ASSY 2).

With the transmitter and the insertion device assembly including the same according to the present disclosure, the following effects can be obtained.
1) First, by configuring the transmitter to be divided and separated into the sensor assembly and the electronic assembly, it is possible to sterilize only the sensor assembly without a separate shielding device.
2) By configuring the transmitter to be divided and separated into the sensor assembly and the electronic assembly, the sensor and needle, which are parts directly inserted into the user's body, and the lower surface of the transmitter and the lower surface of the applicator, which contact the user's skin, are modularized as the first assembly, thereby enabling sterilization of required portions with only one e-beam irradiation.
3) By configuring the transmitter to be divided and separated into the sensor assembly and the electronic assembly, the substrate, on which the electronic components that may risk partial functional loss when directly exposed to e-beam irradiation are mounted, and structures such as the upper housing or the driving portion of the applicator, which substantially require little sterilization, are excluded from the first assembly, and accordingly, there is an advantage in that sterilization is performed by e-beam irradiation without risk of malfunction of the electronic devices mounted on the substrate and sterilization of the insertion device assembly can be achieved within a narrow range and short e-beam irradiation time.
4) By configuring the transmitter such that the sensor assembly and the electronic assembly can be easily coupled through the hook structure, bonding, or the like, the electronic assembly can be easily coupled to the sensor assembly after sterilization of the sensor assembly, so that assembly and sterilization processes can be performed quickly and easily even when the transmitter is configured to be divided and separated into the sensor assembly and the electronic assembly.
5) By configuring the transmitter to be divided and separated into the sensor assembly and the electronic assembly, the lower surface of the transmitter includes two parts, namely, the lower surface of the base portion and the lower surface of the lower case, so that force applied to the substrate accommodated in the transmitter upon firing through the applicator is dispersed and reduced.
6) By arranging the terminals and the electrodes to electrically connect the sensor assembly and the electronic assembly during assembly, the sensor and the substrate can be electrically connected simultaneously with coupling of the sensor assembly and the electronic assembly, without requiring a separate electrical connection work.
7) By configuring the connector that connects the electrodes and the terminals to the sensor in a clip form, the sensor can be electrically connected to the electrodes and the terminals using the connector without a separate configuration for fixing the sensor, and the sensor can also be fixed to the sensor assembly.
8) By the conversion terminal installed in the sensor housing, the mode conversion of the transmitter is possible.

The above description is merely illustrative of the technical idea of the present disclosure, and those of ordinary skill in the art to which the present disclosure pertains will be able to make various modifications and variations without departing from the essential characteristics of the present disclosure. Accordingly, the embodiments described above are not intended to limit the technical idea of the present disclosure, but to explain the technical idea, and the scope of the technical idea of the present disclosure is not limited by these embodiments. The scope of protection of the present disclosure should be interpreted by the following claims, and all technical ideas within the scope equivalent thereto should be construed as being included in the scope of the present disclosure.

### Description of reference numerals

110: upper housing
120: button
130: cap
140: lower housing
150: driving portion
160: needle carrier
171: needle
172, 172a: grip portion
173: protrusion
174: sealing member
200, 200a: transmitter
210, 210a: sensor assembly
211: base portion
212: outer wall
213: fastening hook
214: sensor
215, 215a: electrode portion
216, 216a: contact terminal
217, 217a: connection terminal
218, 218a: internal terminal
219: external terminal
220: holder
221: groove
222, 222a: connector
223, 223a: sensor contact portion
224, 224a: terminal contact portion
225: installation portion
225a: fixing portion
226: installation hook
226a: mounting groove
227, 227a: sensor housing
228: opening
229, 229a: conversion terminal
230: guide protrusion
231, 231a: activation terminal
250, 250a: electronic assembly
251: upper case
252: lower case
253: locking portion
254, 254a: connection electrode
255: substrate
256: battery
257: circuit terminal

## Claims

1. A transmitter, comprising:
a sensor assembly including a base portion, and a sensor that passes through the base portion, wherein a proximal portion of the sensor is positioned at a lower portion of the base portion and a distal portion is positioned at an upper portion of the base portion; and
an electronic assembly accommodated in the upper portion of the base portion of the sensor assembly, wherein the electronic assembly includes, in a state in which the electronic assembly is coupled to the sensor assembly,
a lower case positioned at the upper portion of the base portion, an upper case positioned at an upper portion of the lower case, and a substrate positioned in a space between the upper case and the lower case.

2. The transmitter according to claim 1, wherein the sensor assembly further includes:
an electrode portion including a contact terminal positioned close to the sensor side, and a connection terminal electrically connected to the contact terminal;
a connector electrically connecting the sensor to the contact terminal; and
a sensor housing covering the sensor, the connector, and the contact terminal, and including an opening formed in an upper portion, and
the electronic assembly further includes
a connection electrode positioned in the lower case to electrically connect the connection terminal to a circuit terminal formed on the substrate.

3. The transmitter according to claim 2, wherein the connector includes a sensor contact portion in contact with the sensor, and a terminal contact portion extending from the sensor contact portion to contact the contact terminal.

4. The transmitter according to claim 3, wherein
the sensor assembly further includes an installation portion where the connector is installed on the base portion, and
the connector is installed such that, in a state where one side of the sensor is supported by one side of the installation portion, the sensor contact portion is in contact with other side of the sensor, thereby fixing the sensor.

5. The transmitter according to claim 4, wherein
the installation portion further includes an installation hook formed on one side,
the connector covers an upper portion of the sensor and includes a plurality of bent portions formed therein, and
the connector is fixed to the installation portion by the bent portions being engaged with the installation hook.

6. The transmitter according to claim 2, wherein the connector includes:
a fixing portion including a mounting groove formed therein, wherein a distal portion of the sensor is fitted into the mounting groove; and
a connection pin including a sensor contact portion installed on the fixing portion and positioned on the mounting groove side and a terminal contact portion extending from the sensor contact portion to contact the contact terminal,

7. The transmitter according to claim 6, wherein the connection pin has elasticity, the sensor contact portion of the connection pin is pressed as the distal portion of the sensor is fitted into the mounting groove, and after the sensor is mounted, the sensor contact portion presses the distal portion of the sensor, while the distal portion of the sensor and the contact terminal are electrically connected by the connection pin.

8. The transmitter according to claim 6, further comprising a guide protrusion disposed on an inner surface of the sensor housing to guide a position at which the connector is installed.

9. The transmitter according to claim 2, further comprising a holder positioned on the base portion to surround a portion of the sensor at a position where the sensor is penetrating, wherein an upper portion of the holder closes the opening of the sensor housing.

10. The transmitter according to claim 2, wherein the sensor housing further includes:
a conversion terminal formed therein and contacting the contact terminal; and
an activation terminal electrically connected to the conversion terminal and exposed to exterior of the sensor housing.

11. The transmitter according to claim 2, wherein the electronic assembly further includes:
a conversion terminal formed therein and contacting the substrate; and
an activation terminal electrically connected to the conversion terminal and exposed to exterior of the upper case.

12. An insertion device assembly, comprising:
a transmitter including:
a sensor assembly including a base portion, and a sensor that passes through the base portion, wherein a proximal portion of the sensor is positioned at a lower portion of the base portion and a distal portion is positioned at an upper portion of the base portion;
an electronic assembly that can be accommodated in the upper portion of the base portion of the sensor assembly, wherein the electronic assembly includes, in a state in which the electronic assembly is coupled to the sensor assembly,
a lower case positioned at the upper portion of the base portion, an upper case positioned at an upper portion of the lower case, and a substrate positioned in a space between the upper case and the lower case; and
an applicator having an internal space in which the transmitter is mounted, the applicator including:
an upper housing;
a lower housing coupled to a lower portion of the upper housing and forming an internal space with the housing;
a needle assembly including a grip portion positioned above the transmitter in the internal space, and a needle positioned below the grip portion to pass through the transmitter and surround a portion of a circumference of a proximal portion of the sensor;
a needle carrier disposed in the internal space and configured to linearly move the needle assembly; and
a driving portion that provides power to linearly move the needle assembly.

13. The insertion device assembly according to claim 12, wherein
the sensor assembly further includes:
an electrode portion including a contact terminal positioned close to the sensor side, and a connection terminal electrically connected to the contact terminal;
a connector electrically connecting the sensor to the contact terminal; and
a sensor housing covering the sensor, the connector, and the contact terminal, and including an opening formed in an upper portion, and
the electronic assembly further includes
a connection electrode positioned in the lower case to electrically connect the connection terminal to a circuit terminal formed on the substrate.

14. The insertion device assembly according to claim 13, wherein the connector includes a sensor contact portion in contact with the sensor, and a terminal contact portion extending from the sensor contact portion to contact the contact terminal.

15. The insertion device assembly according to claim 14, wherein
the sensor assembly further includes an installation portion where the connector is installed on the base portion, and
the connector is installed such that, in a state where one side of the sensor is supported by one side of the installation portion, the sensor contact portion is in contact with other side of the sensor, thereby fixing the sensor.

16. The insertion device assembly according to claim 15, wherein
the installation portion further includes an installation hook formed on one side,
the connector covers an upper portion of the sensor and includes a plurality of bent portions formed therein, and
the connector is fixed to the installation portion by the bent portions being engaged with the installation hook.

17. The insertion device assembly according to claim 13, wherein the connector includes:
a fixing portion including a mounting groove formed therein, wherein a distal portion of the sensor is fitted into the mounting groove; and
a connection pin including a sensor contact portion installed on the fixing portion and positioned on the mounting groove side and a terminal contact portion extending from the sensor contact portion to contact the contact terminal,

18. The insertion device assembly according to claim 17, wherein the connection pin has elasticity, the sensor contact portion of the connection pin is pressed as the distal portion of the sensor is fitted into the mounting groove, and after the sensor is mounted, the sensor contact portion presses the distal portion of the sensor, while the distal portion of the sensor and the contact terminal are electrically connected by the connection pin.

19. The insertion device assembly according to claim 17, further comprising a guide protrusion disposed on an inner surface of the sensor housing to guide a position at which the connector is installed.

20. The insertion device assembly according to claim 13, further comprising a holder positioned on the base portion to surround a portion of the sensor at a position where the sensor is penetrating, wherein an upper portion of the holder closes the opening of the sensor housing, and the needle passes through the holder.

21. The insertion device assembly according to claim 13, further comprising a protrusion positioned on a lower portion of the grip portion and passing through the opening of the sensor housing to surround a portion of the sensor.

22. The insertion device assembly according to claim 21, further comprising a sealing member positioned between the protrusion and the opening of the sensor housing, wherein
in a state in which the needle assembly is coupled to the transmitter, an internal space of the grip portion and the sensor housing is sealed by the sealing member.

23. The insertion device assembly according to claim 13, wherein
the sensor housing further includes:
a conversion terminal formed therein and contacting the contact terminal; and
an activation terminal electrically connected to the conversion terminal and exposed to exterior of the sensor housing, and
the applicator further includes a conductor that contacts the activation terminal while the transmitter is in a mounted state.

24. The insertion device assembly according to claim 13, wherein
the electronic assembly further includes:
a conversion terminal formed therein and contacting the substrate; and
an activation terminal electrically connected to the conversion terminal and exposed to exterior of the upper case, and
the applicator further includes a conductor that contacts the activation terminal while the transmitter is in a mounted state.
